# EUROPEAN PATENT SPECIFICATION

(11) **EP 0 835 103 B1**
(45) Date of publication and mention of the grant of the patent: **08.06.2005**
(21) Application number: 96919643.5
(22) Date of filing: 26.04.1996
(51) Int. Cl.: A61K 9/51

(54) **NANOPARTICLES FOR ORAL ADMINISTRATION OF PHARMACEUTICAL AGENTS OF LOW SOLUBILITY**
NANOPARTIKELN FÜR DIE PERORALE VERABREICHUNG VON SCHWERLÖSLICHEN ARZNEIMITTEL
NANOPARTICULES POUR L'ADMINISTRATION ORALE D'AGENTS PHARMACEUTIQUES DE FAIBLE SOLUBILITE

(30) Priority: 08.05.1995 EP 95810306
(43) Date of publication of application: 15.04.1998
(73) Proprietor: Novartis AG, 4056 Basel (CH); Gurny, Robert, 1204 Geneva (CH); Novartis Pharma GmbH, 1230 Wien (AT)
(72) Inventor: GURNY, Robert, CH-1204 Geneva (CH); ALLEMANN, Eric, Olivier, CH-1257 Croix-de-Rozon (CH); LEROUX, Jean-Christophe, Outremont (Québec) H2V 4P1 (CA)
(86) International application number: PCT/EP1996/001769
(87) International publication number: WO 1996/035414

(56) References cited:
- EP-A- 0 275 796
- GB-A- 2 166 651
- US-A- 5 382 435
- JOURNAL OF MICROENCAPSULATION, vol. 8, no. 2, April 1991 - June 1991, GB, pages 161-170, XP000216615 R. BODMEIER ET AL: "Spontaneous formation of drug-containing acrylic nanoparticles"
- EUROPEAN JOURNAL OF PHARMACEUTICS AND BIOPHARMACEUTICS, vol. 39, no. 5, October 1993, DE, pages 173-191, XP000403429 E. ALLEMANN ET AL: "Drug-Loaded Nanoparticles - Preparation Methods and Drug Targeting Issues" cited in the application

## Description

The present invention relates to pharmaceutical compositions for the oral administration of pharmaceutical agents of low water-solubility and to a process for the preparation of said pharmaceutical compositions.

The oral administration of pharmaceutical agents formulated as tablets, capsules, or dragées has certain advantages over parenteral adminstration such as i.v. or i.m.. A certain psychological aspect cannot be ignored. Diseases requiring treatment with "painful" injectable formulations are considered far more "serious" than those diseases being treated with other oral dosage forms. The really important advantage of oral formulations is held to be their suitability for self-administration by the patient as against parenteral formulations that have to be administered in most cases by a physician or paramedical personnel.

An oral dosage form desaggregates after administration and is then subjected to the action of liquids present in the gastrointestinal tract, such as gastric and intestinal juices. A large group of orally administered active agents have lipophilic properties and are, therefore, sparingly soluble in those liquids. This diminishes the amount available for resorption which, in turn, reduces the bioavailability of the active agent administered. To compensate for such loss of bioavailability, the administration of a higher dose is required. However, higher doses of many active agents having low water solubility such as NSAIDs, e.g. acetyl salicylic acid or ibuprofen, or bisphosponates, e. g. sodium pamidronate, are undesirable because of side-effects such as peptic ulceration.

Accordingly, the problem to which the present invention relates may be defined as follows: It is desirable to provide an oral dosage form for active agents of low water solubility in a physiologically acceptable dose. To solve this problem it is necessary to enhance the solubility of the active agent to be administered in the oral dosage form.

Numerous attempts have been made according to the the prior art to increase the solubility of an active agent, especially in fluids present in the gastrointestinal tract (GIT). One approach is the addition of so-called solubility promotors or solubilizers, e.g. hydrophilic cosolvents such as ethanol or propylene glycol, liquid polyethylene glycols, or lipophilic solubilizers such as lecithin, polyglycol esters of fatty acids or polyglycol esters of fatty acid glycerides. The use of those solubilizers generates other problems, for example diminished stability due to phase separation of the individual components of the formulation, or lower gastrointestinal tolerance. Many solubilizers are not acceptable for the incorporation in oral dosage forms.

It the addition of the above-mentioned solubilizers still fails to promote the solubility of the active agent, the incorporation in a homogenous lipid dispersion has been proposed. In such a dispersion the active agent is encapsulated in lipid particles having a particle size smaller than 1 µm. The "loaded" lipid particles then form with the aqueous carrier liquid an aqueous phase of colloidally dispersed or, preferably, finely dispersed character, which differs from the true homogeneous distribution of solutes at molecularty dispersed level but is, nevertheless, sufficiently homogeneous for the preparation of intravenous and oral dosage forms.

Numerous publications suggest the incorporation of active pharmaceutical agents of low solubility in micells, mixed micells, reversed micells, unilamellar or multilamellar liposomes, nanocapsules or nanoparticles.

These methods have the clear advantage of improved solubilization of an active agent having markedly low solubility. Unfortunately, these advantages are again diminished by other problems, including the low stability of the aqueous systems due to the separation of the phase into the invidual components, insufficient amounts of encapsulated active agent, the strong dependency of the particle size on the method and conditions employed, unsatisfactory uniformity and insufficient reproducibility of the products obtained, and other problems.

Surprisingly, it has now been found that selected pharmaceutically acceptable polymers are suitable for the preparation of nanoparticles which encapsulate the active agent of low water-solubility in therapeutically effective amounts and release the active agent in target regions of the gastrointestinal tract.

The present invention relates to a pharmaceutical composition for the oral administration of an active agent having low water-solubility, wherein
a) the active agent is dispersed in an aqueous formulation base; and
b) the solubilizing agent is suitable for the formation of an aqueous dispersion of nanoparticles;
wherein the solubilizing agent is a pharmaceutically acceptable polymer chosen from at least one of (i) a copolymer selected from the group consisting of (a) methacrylic acid or acrylic acid and (b) methyl or ethyl esters of acrylic or methacrylic acid monomers, (ii) polyvinyl acetate phthalate (PVAP) (iii) hydroxypropyl methyl cellulose acetate succinate (HPMCAS), (iv) hydroxypropyl methyl cellulose phthalate (HPMCP), (v) cellulose acetate phthalate (CAP) and, (vi) cellulose acetate trimillitate (CAT),
wherein the polymer is resistant to gastric juices and soluble in intestinal juices.

The pharmaceutical composition according to the present invention has the benefit of providing an enhanced solubility and bioavailability of the active agent to be administered in the oral dosage form. The active agent is released in selected target regions of the gastrointestinal tract such as the small intestine. The release in those regions is desirable in view of improved resorption through larger areas of the epithelium and of larger amounts of juice present in the intestine which reduces the risk of ulceration as compared with gastric resorption.

The general terms used throughout the specification of this invention are preferably defined as follows:
The term pharmaceutical composition means a mixture containing the active agent of low water-solubility to be administered in the oral dosage form to a host in a therapeutic method of treating the disease or condition indicated.

The term oral administration means the enteral administration of a dosage form commonly known as oral dosage form.

Oral dosage forms are in particular solid oral dosage forms containing defined amounts of the active agent, such as capsules or sachets, but also liquid dosage forms, such as droplets, suspensions, or emulsions. Capsules are dry-filled capsules made of gelatin, especially hard gelatin, which are prepared, where appropriate, with the addition of solid excipients, and which are dissolved without time delay by the action of gastric juice to release the components a) and b). Suitable excipients such as sorbitol, lactose, starch, or magnesium stearate, may be admixed. Soft capsules, may contain the liquid dosage forms mentioned, in particular suspensions or emulsions. They may contain, as additives, glycerol, lecithin, fats, oil, paraffin oil or liquid polyethylene glycol. Dry-filied capsule sizes 0-4, preferably 0-2, are suitable, depending on the dose to be administered. Commercial products marketed by Eli Lilly, Elanco, Capsugel, Shionogi, or Scherer are suitable.

Sachets are containers, e.g. bags made of polyethylene, lined paper or aluminum foil, that contain components a) and b) and, optionally, additives such as lactose or starch. The composition may be removed directly after opening the sachet and administered orally, e.g. admixed with water.

An active agent of low water solubility preferably has a water solubility of less than 500 mg/1000 ml, particularly less than 200 mg/1000 ml and may be selected from any therapeutic group such as immune suppressive agents, non-steroidal antiinflammatory agents (NSAID), calcium channel blockers, immunomodulators, antibiotic agents and others.

A particularly preferred active agent having low water solubility is a selected HIV-1 protease inhibitor to which the following formula has been asigned: 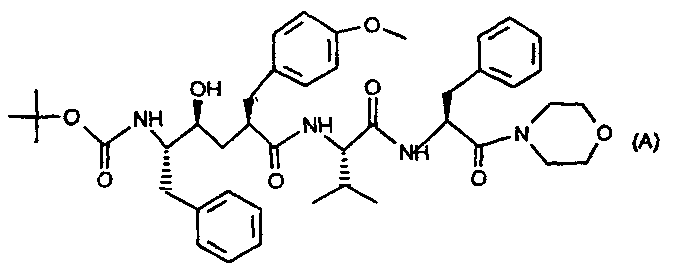 HIV-1 protease was first suggested by Kramer et al. (Science **231,** 1580 - 1584, (1986)) being a target for AIDS treatment. Since then several types of HIV-1 protease inhibitors have become known. Like many other active agents of peptidic structure, this HIV-1 protease inhibitor (A) has a relatively short half-life in some in-vivo pharmaceutical models (mouse) and suffers from an insufficient oral bioavailability. The latter effect is presently attributed to the extremely low aqueous solubility (8 mg/ 1000 ml at pH 7.4).

The following nomenclature has been assigned to the HIV-1 protease inhibitor of the formula (A) given above:
Boc-Phe[C]-(p-CH₃O)Phe-(L)-(Phe-morpholin-4-yl)-amide or 5(S)-tert.-Butoxycarbonylamino-4(S)-hydroxy-2(R)-4-methoxyphenylmethyl-6-phenyl-hexanoyl-(L)-Val-(L)-Phe-morpholin-4-ylamide. This compound is referred to in this specification as HIV-1 protease inhibitor of formula A or, where appropriate, as active agent. The preparation of this compound is described in the Published European Patent Application (EP-A) No. 618 222 (publication date Oct. 5, 1994).

The term "solubilized" means the homogenous dispersion of the active agent having low water solubility in an aqueous phase with the aid of a pharmaceutically acceptable solubilizer which is suitable for the preparation of nanoparticles.

Nanoparticles are solid spheroid particles ranging in size from about 10 to 1000 nm. When dispersed in an aqueous phase, they have colloidal properties. Nanoparticles is a generic term that comprises nanospheres and nanocapsules. Nanospheres have a polymeric matrix type structure, whereas nanocapsules have a shell formed of polymers surrounding an inner liquid core. Nanoparticles encapsulate the active agent of low water solubility.

The term "encapsulate" means the presence of an active agent having low water solubility in nanoparticles. In nanospheres, the active agent may be adsorbed at their surface, or entrapped, e. g. as microcrystals, in the polymeric matrix or dissolved therein. In nanocapsules the active agent may be dispersed in the liquid present in the inner core, but may also be adsorbed at the surface.

The term "aqueous formulation base" means the aqueous carrier liquid wherein the nanoparticles containing the active agent having low water solubility are homogeneously dispersed. The carrier liquid may contain conventional additives customarily used for preparing liquid oral dosage forms which are administered directly in the form of syrups or drops or administered with the aid of small containers such as capsules.

A solubilizing agent which is suitable for the formation of an aqueous dispersion of nanoparticles is, for example, a pharmaceutically acceptable copolymer which is resistant to gastric juice and soluble in intestinal juices. This copolymer inhibits the release of the active agent under strongly acidic conditions present in gastric fluids but allows the controlled release of the active agent (drug targeting) from nanoparticles in pH-neutral or slightly basic juices present in the small intestine. The largest amount of active agent is released in the duodenum, but some release in the jejunum is also possible.

A suitable copolymer, which is resistant to gastric juice and soluble in intestinal juices, is formed from monomers selected from the group consisting of methacrylic acid, methacrylic acid esters, acrylic acid and acrylic acid esters. Those polymers are commercially available from Röhm Pharma GmbH, Weiterstadt Germany marketed under the trademark EUDRAGIT (Registered Trademark of Röhm Pharma GmbH).

An especially preferred polymer is the 1:1- up to 1:2-copolymer which is resistant to gastric juice and soluble in intestinal juices and which is formed from monomers selected from the group consisting of methacrylic acid and methacrylic acid lower alkyl esters, such as the 1:1- up to 1:2-copolymer from methacrylic acid and methyl methacrylate. The 1:1-copolymers are marketed in the EUDRAGIT L series. The corresponding 1:2-copolymers are marketed in the EUDRAGIT S series.

An especially preferred polymer is the 1:1-copolymer of methacrylic acid and acrylic acid ethyl ester. This polymer is marketed under the product name EUDRAGIT L 100-55.

An alternative polymer suitable for the formation of nanoparticles is polyvinyl acetate phthalate (PVAP) or a pharmaceutically acceptable cellulose derivative selected from the group consisting of hydroxypropyl methyl cellulose acetate succinate (HPMCAS), hydroxypropyl methyl cellulose phthalate (HPMCP), cellulose acetate phthalate (CAP), and cellulose acetate trimellitate (CAT).

HPMCP is marketed as aqueous dispersion by Eastman Kodak Corp., HPMCP 50 (USP/NF type 220824) and HPMCP 55 (USP/NF type 200731) are especially preferred.

CAP is marketed as aqueous dispersion under the trademark AQUATERIC (Registered Trademark of FMC Corp.) or is commercially available from Eastman (composition: phthalyl 35 %, acetyl 24 %, moisture 1 %, free acid 0.5 % (as phthalic acid)).

CAT is commercially available from Eastman (composition: trimellityl 29 %, acetyl 22 %, moisture 1 %, free acid 0.5 % (as phthalic acid)).

The present invention also relates process for the preparation of the pharmaceutical composition, which is characterized in that an aqueous dispersion of nanoparticles containing a) the active agent to be solubilized and b) the solubilizing agent, which is suitable for the formation of an aqueous dispersion of nanoparticles, is formed; and the dispersion is processed further under the optional addition of pharmaceutically acceptable additives c), which are suitable for the incorporation in a dosage form for the oral administration.

Various methods for carrying out this process are known. They are compiled in the publication of Eric Allémann et al., Eur. J. Pharm. Biopharm. 39(5), 173 - 191 (1993). The methods for the preparation of nanospheres are particulary preferred.

An especially preferred method comprises the preparation of an aqueous gel containing a hydrophilic polymer under the optional addition of a water soluble salt. This gel is then combined with a solution of a non-toxic organic solvent containing the active agent and the polymer which is suitable for the formation of an aqueous dispersion of nanoparticles. Phase separation is observed, and after addition of water the nanoparticles formed are homogeneously dispersed in the aqueous phase. The aqueous phase is then processed further to the pharmaceutical dosage form intended, e.g. by applying conventional purification and separation methods.

The preparation of the aqueous gel containing the hydrophilic polymer is disclosed in the reference of E. Allemann, loc. cit., and the references cited therein. The gel is formed by the addition of water to the hydrophilic polymer. Suitable hydrophilic polymers are polyvinyl alcohols, such as the ones marketed under the trademark MOWIOL (Registered Trademark of Hoechst AG, Germany). Preferred are polyvinyl alcohols having a degree of hydrolysis of more than 70 % (partially hydrolyzed grades), especially more than 87 %, e.g. MOWIOL from the 88 and 92 series, e.g. 4-88, 5-88, 8-88, 18-88, 23-88, 26-88, and 40-88. To facilitate the phase separation from the organic phase subsequently added, the addition of a physiologically acceptable water-soluble salt, such as magnesium chloride, or magnesium acetate, to the gel phase is preferred.

The gel phase is added under stirring to a solution of a non-toxic organic solvent, e.g. acetone or benzyl alcohol, containing the active agent, e.g. the HIV-1 protease inhibitor of the formula (A) of above, and the pharmaceutically acceptable polymer, which is suitable for the formation of nanoparticles defined above, especially EUDRAGIT from the L and S series, especially EUDRAGIT L 100, L 100-55 or S 100.

Pure water is added to allow the diffusion of the organic solvent to the aqueous phase, and the nanoparticles are formed and homogeneously dispersed therein. The aqueous phase may be processed further by conventional purification and separation methods resulting in the preparation of the dosage form desired.

The dispersion obtained may be defined as aqueous suspension of nanoparticles containing the active agent having low water-solubility. According to the preferred method of phase separation of the aqueous gel from the organic solvent, a homogeneous dispersion of nanospheres is obtained. Nanospheres are clearly distinguishable with physical methods, such as photon correlation spectroscopy (PCS), e. g. with a COULTER NANO-SIZER, LASER light scattering methods, or electron microscopy from other microparticles such as liquid crystals, micells, reversed micells, liposomes, microspheres or microcapsules. For a statistical average of more than 80 %, preferably more than 90 %, a mean average particle size between 60 and 300 nm has been determined. The size of the nanoparticles obtained depends on the established and known methods chosen for their preparation.

The homogenous dispersion containing nanospheres is then processed further to a conventional pharmaceutical dosage form by applying standard purification methods, such as the ones known in the art for purifying nanoparticles, such as ultracentrifugation, cross - flow filtration, or sterile filtration. The dispersion can also be lyophilized in a conventional manner and the lyophilisate is reconstituted to the pharmaceutical dosage form desired.

An oral dosage form is prepared by applying known methods such as the ones mentioned in Hagers Handbuch der Pharmazeutischen Praxis or Remington's Pharmaceutical Sciences. The additives customarily used for the preparation of oral dosage forms may be added if necessary. Their choice depends on the type of dosage form requested, e. g. solid or liquid oral dosage forms.

The homogeneous dispersion containing the nanospheres may also be converted to a lyophilisate which is reconstituted by the addition of water before the dispersion is administered. Even after reconstituting the lyophilisate, a homogenous nanodispersion is formed again. When preparing the lyophilisates, the addition of calculated amounts of water soluble additives is recommended.

The homogeneous dispersion, optionally after concentration to standardized volumes, or the lyophilisate is then added to suitable containers for unitary dosage forms, such as vials.

The following Examples are illustrating the invention as disclosed in the instant specification without limiting the scope thereof; temperatures are given in degrees Celsius; all percentages mentioned are weight percentages (w/w):

### Example 1

1 a) An aqueous gel (42.5 g) containing 60 % magnesium chloride hexahydrate and 11 % polyvinyl alcohol (MOWIOL 4-88, Höchst) is added under stirring (1200 rpm) to an acetone solution (17 g) containing 16.2 % EUDRAGIT S 100 and 1.8 % HIV-1 protease inhibitor of formula A, resulting in the formation of an oil-in-water emulsion. Pure water (50 g) is added to allow the diffusion of acetone into the aqueous phase, with the result of forming monodispersed polymeric nanoparticles.
1 b) The nanoparticulate dispersion is then purified by cross-flow filtration using a SARTOCON Mini Device (Sartorius, Göttingen, Gemany) mounted with a polyolefin cartridge filter with a 100 nm pore size. The filtration procedure is stopped after collecting 10 l of filtrate. The aqueous dispersion is finally frozen for 10 minutes at -55° and freeze-dried for 24 h at 0.05 mbar.
1 c) The lyophilisate is reconstituted in water with gentle agitation. The average particle size measured with a COULTER NANO-SIZER before purification with cross-flow filtration is 264 nm (polydispersity index: 2) and after reconstitution of the lyophilisate is 268 nm (polydispersity index: 3). The freeze-dried nanoparticles contain 9.8 % of the active agent.

### Example 2

An aqueous gel (40 g) containing 14 % polyvinyl alcohol (MOWIOL 4-88, Höchst) is added under stirring (1200 rpm) to a benzyl alcohol solution (17 g) containing 12.8 % EUDRAGIT L 100 and 1.42 % active agent, resulting in the formation of an oil-in-water emulsion. Pure water (660 g) is added to allow the diffusion of benzyl alcohol into the aqueous phase, with the result of forming monodispersed polymeric nanoparticles.

The aqueous dispersion of nanoparticles is purified and freeze-dried for 46 h as described in Example 1 b). The lyophilisate is reconstituted in water with gentle agitation. The average particle size measured with a COULTER NANO-SIZER before purification with cross-flow filtration is 265 nm (polydispersity index: 2) and after reconstitution of the lyophilisate is 271 nm (polydispersity index: 1). The freeze-dried nanoparticles contain 10.0 % of the active agent.

### Example 3

Nanoparticles are prepared, purified and freeze-dried as described above in Example 1a) and 1 b). EUDRAGIT S 100 is replaced with EUDRAGIT L 100-55. The lyophilisate is reconstituted in water with gentle agitation. The average particle size measured with a COULTER NANO-SIZER before purification with cross-flow filtration is 240 nm (polydispersity index: 2) and after reconstitution of the lyophilisate is 246 (polydispersity index: 2). The freeze-dried nanoparticles contain 9.6% of the active agent.

## Claims

1. A pharmaceutical composition for the oral administration of an active agent having low water solubility, wherein
a) the active agent is dispersed in an aqueous formulation base; and
b) the solubilizing agent is suitable for the formation of an aqueous dispersion of nanoparticles;
wherein the solubilizing agent is a pharmaceutically acceptable polymer chosen from at least one of (i) a copolymer selected from the group consisting of (a) methacrylic acid or acrylic acid and (b) methyl or ethyl esters of acrylic or methacrylic acid monomers, (ii) polyvinyl acetate phthalate (PVAP) (iii) hydroxypropyl methyl cellulose acetate succinate (HPMCAS), (iv) hydroxypropyl methyl cellulose phthalate (HPMCP), (v) cellulose acetate phthalate (CAP) and, (vi) cellulose acetate trimellitate (CAT),
wherein the polymer is resistant to gastric juices and soluble in intestinal juices.

2. A pharmaceutical composition according to claim 1 wherein the polymer is a 1:1- or a 1:2-copolymer selected from the group consisting of a (a) methacrylic acid and (b) methyl or ethyl esters of acrylic or methacrylic acid monomers.

3. A pharmaceutical composition according to claim 1 wherein the copolymer is a 1:1- or a 1:2- copolymer of (a) methacrylic acid and (b) methacrylic acid methyl ester.

4. A pharmaceutical composition according to claim 1 wherein the copolymer is a 1:1-copolymer of (a) methacrylic acid and (b) acrylic acid ethyl ester.

5. A pharmaceutical composition according to claim 1 wherein the polymer is hydroxypropyl methyl cellulose phthalate (HPMCP).

6. A pharmaceutical composition according to claim 1 wherein the polymer is cellulose acetate phthalate (CAP).

7. A pharmaceutical composition according to any one of claims 1 to 6 wherein the polymer allows the release of the active agent from the nanopartides in slightly basic juices.

8. A pharmaceutical composition according to any one of claims 1 to 6 wherein the polymer allows the release of the active agent from nanoparticles in pH-neutral juices.

9. A pharmaceutical composition according to any preceding claim, wherein the nanoparticles are nanospheres.

10. A pharmaceutical composition according any preceding claim wherein the active agent has a water solubility of less than 200 mg/1000 ml.

11. A pharmaceutical composition according to any preceding claim wherein the aqueous formulation base contains water soluble additives suitable for incorporation in a dosage form intended for oral administration.

12. A pharmaceutical composition according to any preceding claim wherein the aqueous dispersion of nanoparticles is filled into starch, hard gelatin or soft gelatin capsules.

13. A process for the preparation of a pharmaceutical composition according to claim 1 for oral administration of an active agent having low water solubility comprising
a) preparation of an aqueous gel containing a hydrophilic polymer and optionally a water-soluble salt,
b) preparation of a solution of an organic solvent containing the active agent and the polymer which is resistant to gastric juices and soluble in intestinal juices,
c) combination of the gel obtained in a) with the solution obtained in b), and
d) addition of pure water so that a homogenous aqueous dispersion of nanoparticles is obtained.

14. A process according to claim 13 wherein the hydrophilic polymer is a polyvinyl alcohol.

15. A process according to claim 13 or 14 **characterized in that** the aqueous dispersion of nanoparticles is processed further to a lyophilisate.

## Patentansprüche

1. Pharmazeutische Zusammensetzung zur oralen Verabreichung eines wirksamen Mittels, das eine geringe Wasserlöslichkeit aufweist, wobei
a) das wirksame Mittel dispergiert ist in einem wässrigen Formulierungsgrundstoff; und
b) das lösungsvermittelnde Mittel geeignet ist für die Bildung einer wässrigen Dispersion von Nanopartikeln;
wobei das lösungsvermittetnde Mittel für ein pharmazeutisch annehmbares Polymer steht, das ausgewählt ist aus mindestens einem von (i) einem Copolymer, ausgewählt aus der Gruppe, bestehend aus (a) Methacrylsäure oder Acrylsäure und (b) Methyl- oder Ethylestern von Acryl- oder Methacrylsäuremonomeren, (ii) Polyvinylacetatphthalat (PVAP), (iii) Hydroxypropylmethylcelluloseacetatsuccinat (HPMCAS), (iv) Hydroxypropylmethylcellulosephthalat (HPMCP), (v) Celluloseacetatphthalat (CAP) und (vi) Celluloseacetattrimellitat (CAT),
wobei das Polymer resistent ist gegen Magensäfte und löslich in Darmsäften.

2. Pharmazeutische Zusammensetzung gemäß Anspruch 1, wobei das Polymer für ein 1:1- oder ein 1:2-Copolymer steht, das ausgewählt ist aus der Gruppe, bestehend aus (a) Methacrylsäure und (b) Methyl- oder Ethylestern von Acryl- oder Methacrylsäuremonomeren.

3. Pharmazeutische Zusammensetzung gemäß Anspruch 1, wobei das Copolymer für ein 1:1- oder ein 1:2-Copolymer steht von (a) Methacrylsäure und (b) Methacrylsäuremethylester.

4. Pharmazeutische Zusammensetzung gemäß Anspruch 1, wobei das Copolymer für ein 1:1-Copolymer steht von (a) Methacrylsäure und (b) Acrylsäureethylester.

5. Pharmazeutische Zusammensetzung gemäß Anspruch 1, wobei das Polymer für Hydroxypropylmethylcellulosephthalat (HPMCP) steht.

6. Pharmazeutische Zusammensetzung gemäß Anspruch 1, wobei das Polymer für Celluloseacetatphthalat (CAP) steht.

7. Pharmazeutische Zusammensetzung gemäß einem der Ansprüche 1 bis 6, wobei das Polymer die Freisetzung des wirksamen Mittels aus den Nanopartikeln in leicht basischen Säften erlaubt.

8. Pharmazeutische Zusammensetzung gemäß einem der Ansprüche 1 bis 6, wobei das Polymer die Freisetzung des wirksamen Mittels aus Nanopartikeln in pH neutralen Säften erlaubt.

9. Pharmazeutische Zusammensetzung gemäß einem beliebigen vorstehenden Anspruch, wobei die Nanopartikel für Nanokügelchen stehen.

10. Pharmazeutische Zusammensetzung gemäß einem beliebigen vorstehenden Anspruch, wobei das wirksame Mittel eine Wasserlöslichkeit von weniger als 200 mg/1000 ml aufweist.

11. Pharmazeutische Zusammensetzung gemäß einem beliebigen vorstehenden Anspruch, wobei der wässrige Formulierungsgrundstoff wasserlösliche Additive enthält, die geeignet sind zur Einverleibung in eine Dosierungsform, die zur oralen Verabreichung bestimmt ist.

12. Pharmazeutische Zusammensetzung gemäß einem beliebigen vorstehenden Anspruch, wobei die wässrige Dispersion von Nanopartikeln eingefüllt ist in Stärke-, Hartgelatine- oder Weichgelatinekapseln.

13. Verfahren zur Herstellung einer pharmazeutischen Zusammensetzung gemäß Anspruch 1 zur oralen Verabreichung eines wirksamen Mittels, das eine geringe Wasserlöslichkeit aufweist, umfassend
a) Herstellung eines wässrigen Gels, das ein hydrophiles Polymer und gegebenenfalls ein wasserlösliches Salz enthält,
b) Herstellung einer Lösung eines organischen Lösemittels, das das wirksame Mittel und das Polymer, das resistent ist gegenüber Magensäften und löslich ist in Darmsäften, enthält,
c) Kombination des in a) erhaltenen Gels mit der in b) erhaltenen Lösung, und
d) Zugabe von reinem Wasser, so dass eine homogene, wässrige Dispersion von Nanopartikeln erhalten wird.

14. Verfahren gemäß Anspruch 13, wobei das hydrophile Polymer für einen Polyvinylalkohol steht.

15. Verfahren gemäß Anspruch 13 oder 14, **dadurch gekennzeichnet, dass** die wässrige Dispersion von Nanopartikeln weiter verarbeitet wird zu einem Lyophilisat.

## Revendications

1. Composition pharmaceutique pour l'administration orale d'un agent actif ayant une faible solubilité dans l'eau, dans laquelle
a) l'agent actif est dispersé dans une base de formulation aqueuse; et
b) l'agent solubilisant convient pour la formation d'une dispersion aqueuse de nanoparticules;
dans laquelle l'agent solubilisant est un polymère pharmaceutiquement acceptable choisi parmi au moins l'un des suivants: (i) un copolymère choisi dans le groupe constitué des monomères (a) acide méthacrylique ou acide acrylique et (b) esters méthyliques ou éthyliques d'acide acrylique ou méthacrylique, (ii) le polyacétophtalate de vinyle (PVAP), (iii) l'acétosuccinate d'hydroxypropylméthylcellulose (HPMCAS), (iv) le phtalate d'hydroxypropylméthylcellulose (HPMCP), l'acétophtalate de cellulose (CAP) et l'acétotrimellitate de cellulose (CAT),
le polymère étant résistant aux sucs gastriques et soluble dans les sucs intestinaux.

2. Composition pharmaceutique selon la revendication 1, dans laquelle le polymère est un copolymère 1:1 ou 1:2 choisi dans le groupe constitué des monomères (a) acide méthacrylique et (b) esters méthyliques ou éthyliques d'acide acrylique ou méthacrylique.

3. Composition pharmaceutique selon la revendication 1, dans laquelle le copolymère est un copolymère 1:1 ou 1:2 (a) d'acide méthacrylique et (b) d'ester méthylique d'acide méthacrylique.

4. Composition pharmaceutique selon la revendication 1, dans laquelle le copolymère est un copolymère 1:1 (a) d'acide méthacrylique et (b) d'ester éthylique d'acide acrylique.

5. Composition pharmaceutique selon la revendication 1, dans laquelle le polymère est un phtalate d'hydroxypropylméthylcellulose (HPMCP).

6. Composition pharmaceutique selon la revendication 1, dans laquelle le polymère est un acétophtalate de cellulose (CAP).

7. Composition pharmaceutique selon l'une quelconque des revendications 1 à 6, dans laquelle le polymère permet la libération de l'agent actif à partir des nanoparticules dans des sucs légèrement basiques.

8. Composition pharmaceutique selon l'une quelconque des revendications 1 à 6, dans laquelle le polymère permet la libération de l'agent actif à partir des nanoparticules dans des sucs à pH neutre.

9. Composition pharmaceutique selon l'une quelconque des revendications précédentes, dans laquelle les nanoparticules sont des nanosphères.

10. Composition pharmaceutique selon l'une quelconque des revendications précédentes, dans laquelle l'agent actif a une solubilité dans l'eau inférieure à 200 mg/1000 ml.

11. Composition pharmaceutique selon l'une quelconque des revendications précédentes, dans laquelle la base de formulation aqueuse contient des additifs solubles dans l'eau appropriés pour l'incorporation sous une forme posologique destinée à l'administration orale.

12. Composition pharmaceutique selon l'une quelconque des revendications précédentes, dans laquelle la dispersion aqueuse de nanoparticules est introduite dans des capsules d'amidon, de gélatine dure ou de gélatine molle.

13. Procédé pour la préparation d'une composition pharmaceutique selon la revendication 1, destinée à l'administration orale d'un agent actif ayant une solubilité faible dans l'eau, comprenant
a) la préparation d'un gel aqueux contenant un polymère hydrophile et éventuellement un sel hydrosoluble,
b) la préparation d'une solution d'un solvant organique contenant l'agent actif et le polymère, qui est résistante aux sucs gastriques et soluble dans les sucs intestinaux,
c) la combinaison du gel obtenu en a) avec la solution obtenue en b), et
d) l'addition d'eau pure de manière à obtenir une dispersion aqueuse homogène de nanoparticules.

14. Procédé selon la revendication 13, dans lequel le polymère hydrophile est un alcool polyvinylique.

15. Procédé selon la revendication 13 ou 14, **caractérisé en ce que** la dispersion aqueuse de nanoparticules fait l'objet d'un traitement supplémentaire pour obtenir un lyophilisat.
